# EUROPEAN PATENT SPECIFICATION

(11) **EP 1 341 901 B1**
(45) Date of publication and mention of the grant of the patent: **17.09.2008**
(21) Application number: 01994564.1
(22) Date of filing: 11.12.2001
(51) Int. Cl.: C12N 5/06, C12N 5/08, G01N 33/50

(54) **METHOD FOR IN VITRO CULTURE OF OVARIAN FOLLICLES**
VERFAHREN ZUR IN-VITRO-KULTUR VON OVARIALFOLLIKELN
PROCEDE DE CULTURE IN VITRO DE FOLLICULES OVARIENS

(30) Priority: 13.12.2000 EP 00870300
(43) Date of publication of application: 10.09.2003
(73) Proprietor: VRIJE UNIVERSITEIT BRUSSEL, 1050 Brussel (BE)
(72) Inventor: CORTVRINDT, Rita, B-1840 Londerzeel (BE); SMITZ, Johan, B-1780 Wemmel (BE)
(74) Representative: Van Malderen, Joëlle
(86) International application number: PCT/BE2001/000209
(87) International publication number: WO 2002/048316

(56) References cited:
- US-A- 4 987 080
- SMITZ J ET AL: "OOCYTE IN-VITRO MATURATION AND FOLLICLE CULTURE: CURRENT CLINICAL ACHIEVEMENTS AND FUTURE DIRECTIONS" HUMAN REPRODUCTION, IRL PRESS, OXFORD, GB, vol. 14, no. SUPPL 1, 1999, pages 145-161, XP002901118 ISSN: 0268-1161
- CORTVRINDT R ET AL: "In-vitro maturation, fertilization and embryo development of immature oocytes from early preantral follicles from prepuberal mice in a simplified culture system." HUMAN REPRODUCTION (OXFORD), vol. 11, no. 12, 1996, pages 2656-2666, XP001024487 ISSN: 0268-1161
- SMITZ JOHAN ET AL: "Follicle culture after ovarian cryostorage." MATURITAS, vol. 30, no. 2, 12 October 1998 (1998-10-12), pages 171-179, XP001024462 ISSN: 0378-5122
- PICTON HELEN M ET AL: "In vitro growth of human primordial follicles from frozen-banked ovarian tissue." MOLECULAR AND CELLULAR ENDOCRINOLOGY, vol. 166, no. 1, 15 August 2000 (2000-08-15), pages 27-35, XP002178183 ISSN: 0303-7207
- SMITZ J ET AL: "Improving in vitro maturation of oocytes in the human taking lessons from experiences in animal species." REPRODUCTION IN DOMESTIC ANIMALS, vol. 36, no. 1, February 2001 (2001-02), pages 11-17, XP001073505 ISSN: 0936-6768

## Description

### Field of the invention

The present invention is related to a method for in vitro culture of ovarian follicles. More particularly, the present invention relates to a method for producing large numbers of mature or semi-mature follicles and/or oocytes from the ovarian tissue of a mammal.

### State of the art

Several problems can be identified in the study of folliculogenesis, and the field of oocyte maturation for humans and non-human mammals.

With the widespread acceptance and use of oocyte maturation for animal breeding and for human in vitro fertilisation, large quantities of sperm are commonly collected and banked for future use, in essence creating a limitless supply of sperm. Until now, however, there has existed no practical method for collecting and storing large numbers of fertilisable ova from females. The reason for this relates to the reproductive biology of females. In female mammals, only certain cells in the ovaries are capable of maturing into ova. These germ cells, which are present in a limited amount at birth in all mammals, are held in the ovary, arrested in an early stage of meiosis and incapable of being fertilised at that stage and of developing into normal offspring. Under normal circumstances, a number of these cells begin to develop within the ovary with a periodicity tuned to the animal's reproductive cycle. At the appropriate time in the cycle, either one or a small number of these cells will be released from the ovary, a process known as ovulation. The complex process by which an individual germ cell develops to the point at which ovulation occurs is known as folliculogenesis. Folliculogenesis involves several major steps and the co-ordinated activities of other cells of the ovary as well as pituitary and ovarian hormones. In vivo folliculogenesis is however an inefficient process.

Follicle culture is an experimental technique designed to isolate intact follicles from systemic influences so that their metabolism can be examined scientifically. Using follicle culture, folliculogenesis can be simulated or optimised. However, no follicle culture method is known up until now which can efficiently provide all follicle development stages up to mature follicles and oocytes from primordial follicles. A description of available methods is given in "In vitro culture of ovarian follicles", G.M. Hartshorne, Reviews of Reproduction (1997) 2, 94-104.

Further, almost all successful follicle culture methods up until now use oil to provide a barrier to protect the culture from external influences. This practice limits the possibilities of testing the influence of chemicals (usually xenobiotics) on folliculogenesis, more particularly of lipophilic compounds. Said chemicals can be destructive (negative influence on folliculogenesis) or constructive (positive influence - possible drug detection for treatment of defective folliculogenesis in human or non-human mammals). Such a test could also be devised to assay the influence of physical influences such as electromagnetic or ionising radiation, ultrasound, ... on folliculogenesis, e.g. radiology/radiotherapy. The present state of the art does not allow testing in any discrete stage of folliculogenesis, and usually it is impossible to assess the impact of an external influence on follicle cells in a specific stage of development, as usually a follicle culture tries to mimic the natural situation by including follicles at different stages of development.

Another need that exists is the need for conservation of follicles e.g. in case a human female has to undergo chemotherapy or radiotherapy, or ovariectomy, in vitro fertilisation or implantation with oocytes derived from said conserved follicles would still enable this person to have children from her own oocytes, when this would otherwise be impossible.

It is further clear that such a conservation method for follicles, resulting in a more efficient folliculogenesis combined with artificial insemination would benefit the survival chance of several rare and endangered species.

In other words, there is a need for a new method for generating large numbers of mature or semi-mature follicles and/or oocytes from the ovarian tissue of a mammal.

### Aims of the invention

The present invention aims to provide a novel method for the generation of mature or semi-mature follicles or oocytes from the ovarian tissue of a mammal. More particularly, it is an aim of the invention to provide a method for generating mature or semi-mature follicles or oocytes from the primary or secondary follicles isolated from the ovary tissue of a mammal.

A further aim is to provide a bioassay environment for testing external influences, such as chemicals and physical influences (such as electromagnetic or ionising radiation, ultrasound, ...) on folliculogenesis, and oogenesis more particularly on all steps included in folliculogenesis and oogenesis individually or on follicles and oocytes in a specific developmental stage and isolated from normal systemic influences.

Another aim is to provide a way to generate mature oocytes from conserved ovarian tissue.

### Summary of the invention

The present invention concerns a method for in vitro culture of mammalian ovarian follicles, characterised in that it comprises the following subsequent steps:
- providing a suitable container for the in vitro culture,
- selecting a follicle from an ovary of a mammal, said follicle comprising at least
- a theca or pre-theca cell,
- a granulosa cell and
- an oocyte,
- an optional first culture step using an attachment prohibiting first medium which is free from oil,
- a second culture step using an attachment promoting second medium which is free from oil, and
- obtaining the matured follicle.

Said ovarian follicle can be a primary or a secondary follicle. Further, said ovarian follicle can be a frozen and thawed ovarian follicle, from a stock of ovarian follicles or from ovarian tissue.

In a preferred embodiment, the method of the invention further comprises a third culture step performed after the second culture step using a maturation inducing third medium free from oil, resulting in a mucified cumulus-oocyte complex. Said third step preferably comprises maturation of the oocyte and a ovulation-like shedding of said oocyte.

The optional first culture step preferably comprises the differentiation of primary to secondary follicles.

The second culture step preferably comprises the attachment of the follicle to a surface of the suitable container and the differentiation of the follicle into a preovulatory or preovulatory-like follicle.

Said suitable container is preferably a reduced area 96-well flat bottom culture plate.

Another aspect of the present invention is a method for assaying the effects of a chemical or physical influence on folliculogenesis, comprising the following steps:
- Executing a follicle culture according to the present invention in the presence of said chemical or physical influence during at least one cellular development stage, and
- Assaying the effects.

Applying the chemical or physical influence only at a specified short period during the follicle culture allows assaying the influence at critical points in the follicle's differentiation (e.g. preantral, antral phases, induction of ovulation, ...). Said presence of said chemical or physical influence can also be continuous throughout the duration of the follicle culture.

Said effects can e.g. be conveniently assayed by validation of oocyte quality by a validation method selected from the group consisting of IVF rating, rating of developmental competence after fertilisation and implantation, spindle staining, organelle analysis, chromosome analysis or a combination thereof, or by analysing folliculogenesis quality by a validation method selected from the group consisting of proliferation analysis, differentiation analysis, steroid production, mucification or a combination thereof. The invention is however not linked to the use of these tests to assay oocytes and folliculogenesis quality.

### Short description of the drawings

Fig. 1 represents a schematic overview of a method for generating large numbers of mature or semi-mature follicles and/or oocytes from the ovarian tissue of a mammal according to the present invention.

Figs. 2a and 2b show respectively a follicle in primary and in early preantral (secondary) stage of development.

Figs. 3a and 3b show both a preferred culture plate set-up for practising the method of the present invention.

Figs. 4a to 4e depict the development of follicles cultured according to the method of the present invention.

### Detailed description of the invention

The present invention relates to a method for generating large numbers of mature or semi-mature follicles and/or oocytes from the ovarian tissue of a mammal. More specifically, it relates to methods for the in vitro development and maturation of germ cells recovered from the ovarian tissue of a mammal into mature or semi-mature follicles and/or oocytes.

The present invention will be further clarified using several non-limiting examples.

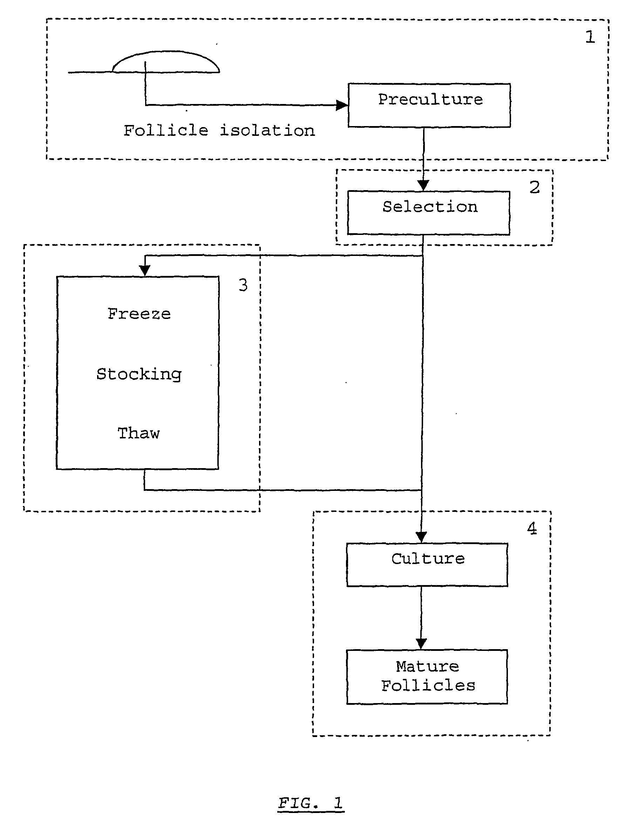

An overview of the method according to the invention can be seen in figure 1, which schematically represents all the necessary steps to obtain mature follicles and oocytes from follicles isolated from the ovarian tissue of a mammal.

All these steps will be further detailed in the following examples:

### Example 1

### Isolation and preculture of secondary ovarian follicles (step. 1 in fig. 1) :

Ovarian tissue is isolated from 12 to 14 days old F1 hybrids C57B1/j6 x CBAca mice and kept in a culture dish comprising Leibovitz's L15 medium with GlutaMAX I^{™} (Gibco BRL 31415-029) with 10% HIA FBS (Heat Inactivated Foetal Bovine Serum) and 0.1 % penicillin-streptomycin-mix (Gibco BRL 3032908). The ovaries have to be freed of oviduct and fat.

With a needle, the surface of the ovaries is scratched, releasing the follicles into suspension into the L15 medium. Under normal conditions, about 30 to 40 suitable follicles per ovary should be obtained with this isolation method.

When one wishes to isolate primary follicles, one can use 7-8 days old mice. About 10-15 suitable follicles per ovary can be obtained in this case.

### Example 2

### Selection of follicles for the method of the invention (step 2 in fig. 1) :

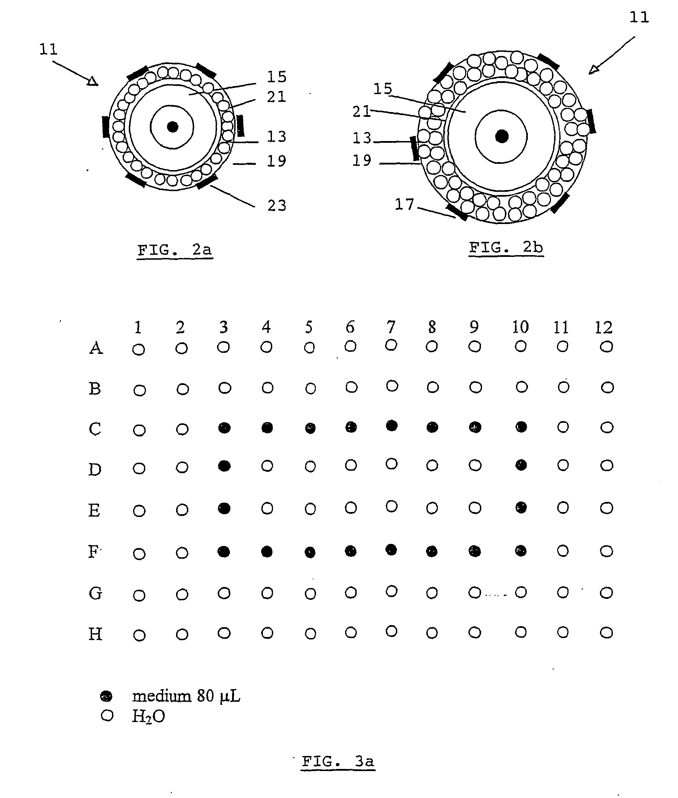

Not all follicles in suspension from example 1 are suitable for follicle culture. To make sure that all follicles grow at approximately the same rate, the selected follicles should have a similar diameter. In this specific case, a diameter of between 100 and 130 µm is suitable and points to development of the follicle up to the early preantral (secondary) stage. In this case, follicles 11 should have two layers of granulosa cells 13 and a round-shaped oocyte 15 as can be seen in fig 2b. Said follicles further comprise theca-interstitial cells 17, a basal membrane 19 and a zona pellucida 21. Follicles are observed under a stereomicroscope and measured under an inverted microscope. Suitable follicles are picked up using suitably arranged pasteur pipettes and transferred to a cryovial (for stocking, see example 3) or to a culture plate (for follicle culture, see example 4).

Follicles in a development stage that is earlier than secondary can also be used to practise the present invention. The most important factor in deciding whether follicles are sufficiently differentiated is the presence of theca cells 17 or pre-theca cells 23, which are present at the outside surface of a primary stage follicle as can be seen in fig 2a. Pre-theca cells might not express the LH receptor yet on their surface. In the mouse model used in the examples, the size of such primary stage follicles lies between about 70 and 100µm. This size is however specific for the mouse system, appropriate size for other species can be determined by the person skilled in the art by relating to the cellular development stage described higher.

### Example 3

### Creation and use of a follicle stock (step 3 in fig. 1):

A follicle stock can be created using cryopreservation techniques known to the skilled person. An example of such a technique is described in the following paragraphs.

Follicles as obtained in Example 2 are collected in plastic cryovials (Simport, Quebec, Canada). 25 follicles per vial are suspended in 150µl of L15 medium with 10% heat-inactivated FCS and 1.5 M DMSO. Slow freezing is performed using a controlled programmed freezing machine (Cell Freezer R204; Planer, Sunbury-on-Thames, UK). Follicles are equilibrated in the freezing mixture for 15 minutes at 4°C and then cooled to -7°C at a rate of 2°C/min. After manual seeding, the temperature is lowered to -40°c at a rate of -0.3°C/min. before storage in liquid nitrogen, the follicles are very rapidly cooled to -110°C at a rate of -50°C/min.

Follicles are thawed ultra-rapidly by warming the cryovials to 37°C. Dilution of the cyoprotectant was done in three steps (reducing the concentration of DMSO from 1.5 M to 1 M and then to 0.5 M) of 15 minutes at room temperature. Before culture, follicles are equilibrated for 15 min in the isolation medium at 37°C From this point on, the follicles can be handled as freshly obtained follicles.

### Example 4

### Follicle culture (step 4 in fig. 1):

The follicle culture comprises of at least one culture step which can provide differentiated follicles. Differentiation is provoked by using suitable media. In the method of the present invention, media can be divided in three groups: a first medium, second medium and third medium. Utilisation of the right medium at the right differentiation stage is crucial for successfully implementing the method of the present invention.

Two examples will illustrate how one can use the method of the present invention with follicles at different differentiation stages.

When referring to incubation periods, the following conditions apply except when otherwise indicated:
- Temperature: 37°C
- Air mixture: 5% CO₂ in air.
- Humidity: 100% saturated

Also, the incubation periods may vary to obtain equivalent results when using other species than mouse. The examples are optimised for the mouse system.

An overview of the functions of the specific media as well as an example of their composition can be seen in the following table:

| Function | First medium | Second medium | Third medium |
|---|---|---|---|
| Differentiation | From pre-theca to theca cell comprising follicle | From secondary follicle to preovulatory (PO) follicle | From PO follicle to in vitro ovulated metaphase II or mature oocyte |
| | (From primary to secondary follicle) | | |
| Fixation | Inhibited fixation to the container surface | Promoted fixation to the container surface | Release of a mucified cumulus-oocyte complex |
| Example | α-mem (Gibco BRL 32571-028) | α-mem (Gibco BRL 32571-028) | α-mem (Gibco BRL 32571-028) |
| composition: | 1% FCS | 5% FCS | 5% FCS |
| | ITS | ITS | ITS |
| | FSH | FSH | FSH |
| | LH | LH | LH |
| | | | HCG |
| | | | EGF |

| | | | |
|---|---|---|---|
| FCS = Foetal Calf Serum, ITS = Insulin-transferrin sodium selenite (5µ/g/5µg/5ng per ml), FSH = Follicle Stimulating Hormone (10 mIU/ml-100 mIU/ml), LH = lutheinising hormone (0-10 mIU/ml), HCG = human chorionic gonadotrophin (1.5 IU/ml), and EGF = Epidermal Growth Factor (10 ng/ml) The α-mem can be replaced by any suitable basal cell culture medium, such as Waymouth medium. | | | |

### 4.A.: example using primary follicles

In the case primary follicles (i.e. follicles with pre-theca cells which have not yet differentiated fully to theca cells and might not express LH receptors on their surface) are used, a first culture step with a first culture medium is necessary.

In this step, the above mentioned first medium is used. This medium will allow the pre-theca cells to grow and further differentiate to theca cells. Follicles are e.g. cultured in 75 µl of medium for a period of 4 days. After 4 days, follicles with a starting size of between 90 and 100 µm will usually reach the secondary stage (preantral stage) of their development (smaller size primary follicles will need up to 8 days to reach the preantral stage). Follicle size will then be about 100 - 130 µm, the increase in size mainly due to granulosa cell proliferation and oocyte growth. From then on the further procedure is the same as when starting from secondary follicles.

### 4.B.: example using secondary follicles

When starting from secondary follicles, one can start immediately with culture using the second medium. This medium will promote adhesion to the culture recipient surface. Culture medium is refreshed every four days. On day 12, the culture medium is refreshed using the third medium in stead of the second. This medium will cause the release of a mucified oocyte-cumulus complex from the follicle overnight, while the oocyte finalises maturation.

Preferably, a 96-well reduced surface area plate (A2, Costar) is used for practising the method of the invention. Cultures are done in 75µl of culture medium. When refreshing medium, 30µl of used medium is removed and 30µl of fresh medium is added. Schematically, both procedures can be summarised as follows:

| Day | Primary | Secondary |
|---|---|---|
| 0 | Plating (medium 1) | Plating (medium 2) |
| 4 | Remove 30 µl | Remove 30 µl |
| | Add 30 µl of medium 2 | Add 30 µl of medium 2 |
| 8 | Remove 30 µl | Remove 30 µl |
| | Add 30 µl of medium 2 | Add 30 µl of medium 2 |
| 12 | Remove 30 µl | Remove 30 µl |
| | Add 30 µl of medium 2 | Add 30 µl of medium 3 |
| 13 | - | Retrieval oocyte |
| 16 | Remove 30 µl | |
| | Add 30 µl of medium 3 | |
| 17 | Retrieval oocyte | |

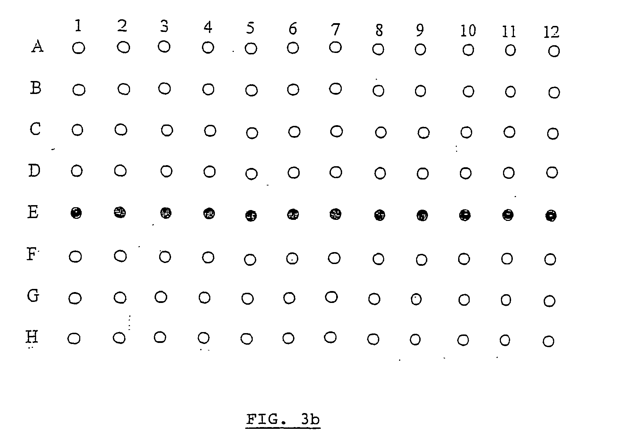

A preferred set-up is presented in fig 3. 96-well reduced surface area flat bottom culture plates (Costar) are used. Fig 3a shows a first possible set-up. Black circles indicate culture wells, white circles designate wells filled with water.

Another possible set-up is given in fig 3b. Here, row E is used for culturing, while all other wells are filled with water. The water is added to create a suitable microenvironment (100% humidity) at plate level.

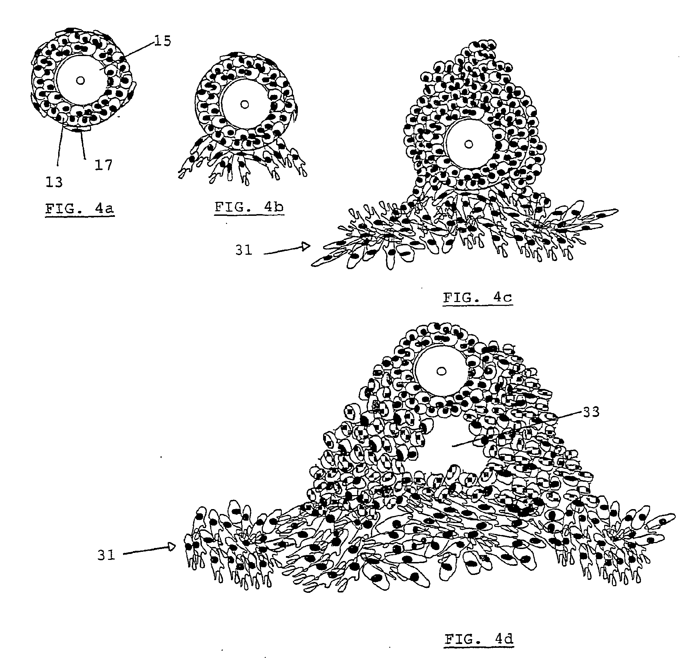

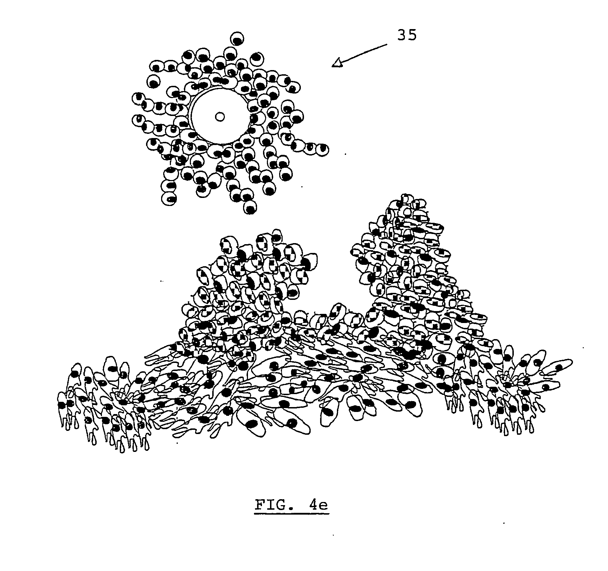

Fig. 4 shows the development of the follicle when using the method according to the present invention. Primary or secondary follicles (a) are brought into culture. When being cultured in the second medium, attachment to the culture vessel surface is promoted. Theca-interstitial cell proliferation (31) is promoted, which results in attachment to the recipient's surface (b,c). During the 12-day culture, the follicle further develops and grows (b,c,d). Growth of the follicle leads to rupture of the structure and the formation of intrafollicular cavities (d) (33). Said cavities and ruptures provide for good medium circulation, thus augmentation of the survival chances for the oocyte, as it will be sufficiently supplied with nutrients and oxygen. Addition of the third medium will lead to release of the oocyte-cumulus complex (e) (35) .

### Example 5

### Use of follicles obtained according to the invention for testing the chemical impact of taxol:

The same method is used as in example 4. Secondary follicles are used. 10 96-well plates with follicle cultures in row E are used for testing the influence of taxol on folliculogenesis (75 µl per well). All media used in the culture steps are constituted as follows:

| Plate number | Taxol concentration |
|---|---|
| 1 and 2 | - (control) |
| 3 and 4 | 0.01 nM |
| 5 and 6 | 0.1 nM |
| 7 and 8 | 1 nM |
| 9 | 10 nM |

The culture medium is refreshed every 4 days by removing 30 µl and addition of 30µl of fresh medium.

After 13 days, follicles are scored on survival, mucification, presence of polar body (PB), and oocyte diameter. Mean values with standard deviations (sd) are given:

| Plate number | Survival | | Mucification | | PB | | diameter | |
|---|---|---|---|---|---|---|---|---|
| | mean | Sd | mean | Sd | mean | Sd | mean | Sd |
| 1 + 2 | 100 % | 0 | 96 % | 0.06 | 78% | 0.07 | 72.9 | 0.57 |
| 3 + 4 | 87 % | 0.19 | 100 % | 0 | 75% | 0 | 72.2 | 0.42 |
| 5 + 6 | 100 % | 0 | 96 % | 0.06 | 75% | 0.11 | 71.6 | 0.14 |
| 7 + 8 | 92 % | 0 | 96 % | 0.06 | 55 % | 0.13 | 71.7 | 0.14 |
| 90 | % | - | 0 | - | 0 | - | 0 | - |

Using this method, the influence of a compound on folliculogenesis and oocyte maturation can be assayed.

### Example 6

### In Vitro Fertilisation (IVF) and embryo transfer (ET) using oocytes obtained with the present invention:

The medium used for IVF comprises KSOM, comprising the following constituents in water:

| | Conc (mM) | Conc (g/l) | Conc (ml/l) |
|---|---|---|---|
| NaCl | 95.00 | 5.552 | |
| KCl | 2.50 | 0.185 | |
| Glucose | 5.56 | 1 | |
| KH₂PO₄ | 0.35 | 0.047 | |
| MgSO₄ | 0.20 | 0.24 | |
| Lactate | 10.00 | | 2.270 |
| Pyruvate | 0.20 | 0.022 | |
| NaHCO₃ | 25.00 | 2.1 | |
| CaCl₂.2H₂O | 1.71 | 0.25 | |
| L-Glutamine | 1.0 | 0.146 | |
| EDTA | 0.01 | 0.0029 | |
| Streptomycin | | 0.05 | |
| Penicillin | | 0.06 | |
| Phenolred | | 0.01 | |

The KSOM medium is supplemented with 3% non-crystalline Bovine Serum Albumin (BSA).

Fully matured oocytes comprising cumulus formation, as obtained in example 4, are selected. Sperm is collected by epididymus removal and dissection. Sperm is then capacitated by incubation in KSOM + 3% BSA for two hours, counted and scored for motility. If necessary, concentration is adjusted to the target concentration of 2.10⁶/ml.

The selected oocyte-cumulus complexes are transferred into 30 µl of KMSO + 3% BSA, and 10 µl of capacitated sperm is added, and an incubation step of 2:30h follows.

After the incubation step, oocytes are transferred to embryo culture dishes (KSOM + 0.5% BSA). Oocytes are first cleaned by repeatedly pipetting with a 20 µl pipette, resulting in detachment of the cumulus cells and the sperm cells from the oocyte.

After 24 hours of further incubation, oocytes can be scored for fertilisation by detection of two-cell embryonal stage.

Fertilisation rates of up to 72 % and blastocyst development rates of up to 90% have been obtained with oocytes provided by the method of the present invention.

## Claims

1. A method for in vitro culture of mammalian ovarian follicles, **characterised in that** it comprises the following subsequent steps:
- providing a suitable container for the in vitro culture,
- selecting a follicle recovered from an ovary of a mammal, said follicle comprising at least
- a theca or pre-theca cell,
- a granulosa cell and
- an oocyte,
- a culture step using an attachment promoting medium which is free from oil, and
- obtaining the matured follicle.

2. The method of claim 1, **characterised in that** said ovarian follicle is a secondary follicle.

3. The method of claims 1 or 2, **characterised in that** the culture step using an attachment promoting medium comprises the attachment of the follicle to a surface of the suitable container and the differentiation of the follicle into a preovulatory or preovulatory-like follicle.

4. The method of any of claims 1 or 3, **characterised in that** the method comprises a primary culture step executed before the culture step using the attachment promoting medium, said primary culture step using an attachment prohibiting first medium, which is free from oil.

5. The method of claim 4, **characterised in that** said ovarian follicle is a primary follicle.

6. The method of claim 4 or 5, **characterised in that** the primary culture step comprises the differentiation of primary to secondary follicles.

7. The method as in any of the claims 1 to 6, **characterised in that** it comprises a further culture step performed after the culture step using the attachment promoting medium, said further culture step using a maturation inducing medium free from oil resulting in a mucified cumulus-oocyte complex.

8. The method as in claim 7, **characterised in that** the further culture step comprises maturation of the oocyte and a ovulation-like shedding of said oocyte.

9. The method of any of the claims 1 to 8, **characterised in that** said ovarian follicle is a frozen and thawed ovarian follicle.

10. The method of any of claims 1 to 9, further **characterised in that** said suitable container is a reduced area 96-well flat bottom culture plate.

11. A method for assaying the effects of a chemical or physical influence on folliculogenesis, comprising the following steps:
• Executing a follicle culture as recited in claim 1 in the presence of said chemical or physical influence during at least one cellular development stage, and
• Assaying the effects.

12. A method as in claim 11, **characterised in that** said effects are assayed by validation of oocyte quality by a validation method selected from the group consisting of IVF rating, rating of developmental competence after fertilisation and implantation, spindle staining, organelle analysis, chromosome analysis or a combination thereof.

13. A method as in claim 11, **characterised in that** said effects are assayed by analysing folliculogenesis quality by a validation method selected from the group consisting of proliferation analysis, differentiation analysis, steroid production, mucification or a combination thereof.

14. A method as in any of the claims 11 to 13, further **characterised in that** said presence of said chemical or physical influence is continuous or discontinuous throughout the duration of the follicle culture.

## Patentansprüche

1. Verfahren zur In-vitro-Kultur von Ovarialfollikeln von Säugern, **dadurch gekennzeichnet, dass** es die folgenden, sich anschließenden Schritte umfasst:
- Bereitstellen eines geeigneten Behälters für die In-vitro-Kultur,
- Auswählen eines Follikels, das aus einem Eierstock von einem Säugetier gewonnen wurde, wobei das Follikel mindestens
- eine Thekazelle oder eine Thekazell-Vorläuferzelle,
- eine Granulosazelle und
- eine Oozyte umfasst,
- einen Kultivierungsschritt unter Verwendung eines Mediums, das die Anheftung fördert, welches frei von Öl ist, und
- Erhalten des reifen Follikels.

2. Verfahren nach Anspruch 1, **dadurch gekennzeichnet, dass** das Ovarialfollikel ein sekundäres Follikel ist.

3. Verfahren nach den Ansprüchen 1 oder 2, **dadurch gekennzeichnet, dass** der Kultivierungsschritt unter Verwendung eines Mediums, welches die Anheftung fördert, die Anheftung von dem Follikel an eine Oberfläche von dem geeigneten Behälter und die Differenzierung von dem Follikel in ein präovulatorisches oder ein präovulatorisch-ähnliches Follikel umfasst.

4. Verfahren nach einem der Ansprüche 1 oder 3, **dadurch gekennzeichnet, dass** das Verfahren einen Primärkulturschritt umfasst, der vor dem Kultivierungsschritt ausgeführt wird, welcher das die Anheftung fördernde Medium verwendet, wobei der Primärkulturschritt ein erstes Medium verwendet, das die Anheftung verhindert und welches frei von Öl ist.

5. Verfahren nach Anspruch 4, **dadurch gekennzeichnet, dass** das Ovarialfollikel ein primäres Follikel ist.

6. Verfahren nach Anspruch 4 oder 5, **dadurch gekennzeichnet, dass** der Primärkulturschritt die Differenzierung der primären Follikel zu sekundären Follikeln umfasst.

7. Verfahren nach einem der Ansprüche 1 bis 6, **dadurch gekennzeichnet, dass** es einen weiteren Kultivierungschritt umfasst, der nach dem Kultivierungsschritt durchgeführt wird, welcher das die Anheftung fördernde Medium verwendet, wobei der weitere Kultivierungsschritt ein die Reifung induzierendes Medium verwendet, das frei von Öl ist und in einem mit einer extrazellulären Matrix umgebenen Cumulus-Oozyten-Komplex resultiert.

8. Verfahren nach Anspruch 7, **dadurch gekennzeichnet, dass** der weitere Kultivierungsschritt die Reifung der Oozyte und ein ovulationsartiges Shedding der Oozyte umfasst.

9. Verfahren nach einem der Ansprüche 1 bis 8, **dadurch gekennzeichnet, dass** das Ovarialfollikel ein gefrorenes und aufgetautes Ovarialfollikel ist.

10. Verfahren nach einem der Ansprüche 1 bis 9, **dadurch gekennzeichnet, dass** der geeignete Behälter eine 96-Well Mikrotiter-Kulturplatte mit flachem Boden ist.

11. Verfahren zur Untersuchung der Wirkungen von einer Chemikalie oder von einer physikalischen Einwirkung auf die Follikelgenese, umfassend die folgenden Schritte:
• Durchführen einer Follikelkultur, wie in Anspruch 1 aufgeführt, in der Gegenwart von der chemischen oder der physikalischen Einwirkung während mindestens eines zellulären Entwicklungsstadiums, und
• Untersuchen der Wirkungen.

12. Verfahren nach Anspruch 11, **dadurch gekennzeichnet, dass** die Wirkungen durch die Überprüfung der Qualität der Oozyten mittels eines Überprüfungsverfahrens untersucht werden, das aus der aus der Beurteilung der In-vitro-Fertilisation, der Beurteilung der Entwicklungskompetenz nach der Fertilisation und Implantation, der Anfärbung des Spindelapparates, der Organellenanalyse, der Chromosomenanalyse oder einer Kombination daraus bestehenden Gruppe ausgewählt ist.

13. Verfahren nach Anspruch 11, **dadurch gekennzeichnet, dass** die Wirkungen durch die Analyse der Qualität der Follikelgenese mittels eines Überprüfungsverfahrens untersucht werden, welches aus der aus Proliferationsanalyse, Differenzierungsanalyse, Steroidproduktion, Muzifikation oder einer Kombination daraus bestehenden Gruppe ausgewählt ist.

14. Verfahren nach einem der Ansprüche 11 bis 13, **dadurch gekennzeichnet, dass** die Gegenwart von der chemischen oder der physikalischen Einwirkung während der Zeitdauer der Follikelkultur kontinuierlich oder diskontinuierlich ist.

## Revendications

1. Procédé de culture *in vitro* de follicules ovariens de mammifère, **caractérisé en ce qu'**il comprend les étapes ultérieures suivantes :
- mettre à disposition un conteneur approprié pour la culture *in vitro,*
- sélectionner un follicule récupéré d'un ovaire d'un mammifère, ledit follicule comprenant au moins
- une cellule thécale ou pré-thécale,
- une cellule de la granulosa et
- un ovocyte,
- réaliser une étape de culture au moyen d'un milieu favorisant un attachement qui est exempt d'huile, et
- obtenir le follicule mature.

2. Procédé selon la revendication 1, **caractérisé en ce que** ledit follicule ovarien est un follicule secondaire.

3. Procédé selon la revendication 1 ou 2, **caractérisé en ce que** l'étape de culture au moyen d'un milieu favorisant un attachement comprend l'attachement du follicule à une surface du conteneur approprié et la différenciation du follicule en un follicule préovulatoire ou en un follicule analogue à un follicule préovulatoire.

4. Procédé selon l'une quelconque des revendications 1 ou 3, **caractérisé en ce que** le procédé comprend une étape de culture primaire exécutée avant l'étape de culture utilisant le milieu favorisant un attachement, ladite étape de culture primaire utilisant un premier milieu ne permettant pas d'attachement, qui est exempt d'huile.

5. Procédé selon la revendication 4, **caractérisé en ce que** ledit follicule ovarien est un follicule primaire.

6. Procédé selon la revendication 4 ou 5, **caractérisé en ce que** l'étape de culture primaire comprend la différenciation de follicules primaires en follicules secondaires.

7. Procédé selon l'une quelconque des revendications 1 à 6, **caractérisé en ce qu'**il comprend une étape de culture supplémentaire réalisée après l'étape de culture utilisant le milieu favorisant un attachement, ladite étape de culture supplémentaire utilisant un milieu exempt d'huile qui induit une maturation donnant lieu à un complexe cumulus-ovocyte présentant une mucification.

8. Procédé selon la revendication 7, **caractérisé en ce que** l'étape de culture supplémentaire comprend la maturation de l'ovocyte et un détachement analogue à l'ovulation dudit ovocyte.

9. Procédé selon l'une quelconque des revendications 1 à 8, **caractérisé en ce que** ledit follicule ovarien est un follicule ovarien congelé et décongelé.

10. Procédé selon l'une quelconque des revendications 1 à 9, en outre **caractérisé en ce que** ledit conteneur approprié est une plaque de culture de surface réduite à fond plat avec96 puits.

11. Procédé pour analyser les effets d'une influence chimique ou physique sur la folliculogenèse, comprenant les étapes suivantes:
• Exécuter une culture de follicule tel que décrit dans la revendication 1 en présence de ladite influence chimique ou physique pendant au moins un stade de développement cellulaire, et
• Analyser les effets.

12. Procédé selon la revendication 11, **caractérisé en ce que** lesdits effets sont analysés par une validation de la qualité de l'ovocyte au moyen d'un procédé de validation sélectionné dans le groupe comprenant l'évaluation d'une FIV, une évaluation des compétences développementales après fertilisation et implantation, la coloration du fuseau, une analyse des organelles, une analyse chromosomique ou une combinaison de ceux-ci.

13. Procédé selon la revendication 11, **caractérisé en ce que** lesdits effets sont analysés par une analyse de la qualité de la folliculogenèse au moyen d'un procédé de validation sélectionné dans le groupe comprenant l'analyse de la prolifération, analyse de la différenciation, production de stéroïdes, mucification ou une combinaison de ceux-ci.

14. Procédé selon l'une quelconque des revendications 11 à 13, en outre **caractérisé en ce que** ladite présence de ladite influence chimique ou physique est continue ou discontinue pendant la durée de culture du follicule.
